# EUROPEAN PATENT APPLICATION

(11) **EP 2 955 225 A1**
(43) Date of publication of application: **16.12.2015**
(21) Application number: 14172216.5
(22) Date of filing: 12.06.2014
(51) Int. Cl.: C12N 9/88, C12P 13/00

(54) **(R)-selective hydroxynitrile lyase variants with a cupin fold having improved substrate scope and the use thereof**

(71) Applicant: ACIB GmbH, 8010 Graz (AT); Technische Universität Graz, 8010 Graz (AT)
(72) Inventor: Wiedner, Romana, 8323 St. Marein bei Graz (AT); Steiner, Kerstin, 8010 Graz (AT); Glieder, Anton, 8200 Hofstätten an der Raab (AT); Schwab, Helmut, 8043 Graz (AT)
(74) Representative: Gassner, Birgitta

(57) **Abstract**

The present invention relates to an improved recombinant cupin-hydroxynitrile lyase (HNL), which is capable to catalyze the asymmetric cyanohydrin reaction, wherein the recombinant cupin-HNL comprises at least one amino acid substitution, preferably at least three substitutions.

SEQUENCE LISTING

<110>  ACIB GmbH
       Technische Universität Graz

<120>  (R)-SELECTIVE HYDROXYNITRILE LYASE VARIANTS WITH A CUPIN FOLD AND
        THE USE THEREOF

<130>  AC008EP

<160>  4

<170>  PatentIn version 3.5

<210>  1
<211>  131
<212>  PRT
<213>  artificial sequence

<220>
<223>  GtHNL

<400>  1

Met Glu Ile Lys Arg Val Gly Ser Gln Ala Ser Gly Lys Gly Pro Ala
1               5                   10                  15

Asp Trp Phe Thr Gly Thr Val Arg Ile Asp Pro Leu Phe Gln Ala Pro
            20                  25                  30

Asp Pro Ala Leu Val Ala Gly Ala Ser Val Thr Phe Glu Pro Gly Ala
        35                  40                  45

Arg Thr Ala Trp His Thr His Pro Leu Gly Gln Thr Leu Ile Val Thr
    50                  55                  60

Ala Gly Cys Gly Trp Ala Gln Arg Glu Gly Gly Ala Val Glu Glu Ile
65                  70                  75                  80

His Pro Gly Asp Val Val Trp Phe Ser Pro Gly Glu Lys His Trp His
                85                  90                  95

Gly Ala Ala Pro Thr Thr Ala Met Thr His Leu Ala Ile Gln Glu Arg
            100                 105                 110

Leu Asp Gly Lys Ala Val Asp Trp Met Glu His Val Thr Asp Glu Gln
        115                 120                 125

Tyr Arg Arg
    130

<210>  2
<211>  131
<212>  PRT

<213>  artificial sequence

<220>
<223>  GtHNL-A40H/V42T/Q110H

<400>  2

Met Glu Ile Lys Arg Val Gly Ser Gln Ala Ser Gly Lys Gly Pro Ala
1               5                   10                  15

Asp Trp Phe Thr Gly Thr Val Arg Ile Asp Pro Leu Phe Gln Ala Pro
            20                  25                  30

Asp Pro Ala Leu Val Ala Gly His Ser Thr Thr Phe Glu Pro Gly Ala
        35                  40                  45

Arg Thr Ala Trp His Thr His Pro Leu Gly Gln Thr Leu Ile Val Thr
    50                  55                  60

Ala Gly Cys Gly Trp Ala Gln Arg Glu Gly Gly Ala Val Glu Glu Ile
65                  70                  75                  80

His Pro Gly Asp Val Val Trp Phe Ser Pro Gly Glu Lys His Trp His
                85                  90                  95

Gly Ala Ala Pro Thr Thr Ala Met Thr His Leu Ala Ile His Glu Arg
            100                 105                 110

Leu Asp Gly Lys Ala Val Asp Trp Met Glu His Val Thr Asp Glu Gln
        115                 120                 125

Tyr Arg Arg
    130

<210>  3
<211>  131
<212>  PRT
<213>  artificial sequence

<220>
<223>  AcHNL

<400>  3

Met Gln Ile Thr Arg Asn Gly Ser Gln Pro Ser Gly Arg Gly Pro Ala
1               5                   10                  15

Glu Tyr Phe Thr Gly Thr Val Arg Val Asp Pro Leu Phe Ala Ala Pro
            20                  25                  30

Glu Pro Ala Arg Val Ala Gly Ala Ser Val Thr Phe Glu Pro Gly Ala
        35                  40                  45

Arg Thr Ala Trp His Thr His Pro Leu Gly Gln Thr Leu Ile Val Thr
    50                  55                  60

Ser Gly Cys Gly Arg Val Gln Arg Glu Gly Gly Pro Val Glu Glu Ile
65                  70                  75                  80

Arg Pro Gly Asp Val Val Trp Phe Thr Pro Gly Glu Arg His Trp His
                85                  90                  95

Gly Ala Ser Pro Ser Thr Ala Met Thr His Ile Ala Ile Gln Glu Lys
            100                 105                 110

Leu Asp Gly Lys Val Val Glu Trp Leu Glu His Val Thr Asp Ala Glu
        115                 120                 125

Tyr Ala Gly
    130

<210>  4
<211>  131
<212>  PRT
<213>  artificial sequence

<220>
<223>  AcHNL-A40H/V42T/Q110H

<400>  4

Met Gln Ile Thr Arg Asn Gly Ser Gln Pro Ser Gly Arg Gly Pro Ala
1               5                   10                  15

Glu Tyr Phe Thr Gly Thr Val Arg Val Asp Pro Leu Phe Ala Ala Pro
            20                  25                  30

Glu Pro Ala Arg Val Ala Gly His Ser Thr Thr Phe Glu Pro Gly Ala
        35                  40                  45

Arg Thr Ala Trp His Thr His Pro Leu Gly Gln Thr Leu Ile Val Thr
    50                  55                  60

Ser Gly Cys Gly Arg Val Gln Arg Glu Gly Gly Pro Val Glu Glu Ile
65                  70                  75                  80

Arg Pro Gly Asp Val Val Trp Phe Thr Pro Gly Glu Arg His Trp His
                85                  90                  95

Gly Ala Ser Pro Ser Thr Ala Met Thr His Ile Ala Ile His Glu Lys
            100                 105                 110

Leu Asp Gly Lys Val Val Glu Trp Leu Glu His Val Thr Asp Ala Glu
        115                 120                 125

Tyr Ala Gly
    130

## Description

### Field of the Invention

The present invention relates to an improved recombinant cupin-hydroxynitrile lyase (HNL), which is capable to catalyze the asymmetric cyanohydrin reaction, wherein the recombinant cupin-HNL comprises at least one amino acid substitution.

### Background Art

Biocatalytic processes have become very important to the chemical industry. Of particular importance is the use of enzymes, when the properties of biocatalysts enable either of the two enantiomers in chemical reaction with chiral or prochiral compounds to be reacted or formed preferentially.

Essential requirements for utilizing these favorable properties of enzymes are their low-cost availability in sufficient amounts, as required in industrial processes, a sufficiently high reactivity, selectivity and high stability under the realistic conditions of the industrial process.

Hydroxynitrile lyases (HNL) catalyze the stereoselective cleavage of cyanohydrins. More importantly, they are valuable tools in biocatalysis, due to their ability to synthesize chiral α-cyanohydrins by a C-C bond forming condensation reaction. Enantiopure cyanohydrins are versatile building blocks and intermediates that serve as starting material for many enzymatic and chemical follow-up reactions, which find application in pharmaceutical, agrochemical and cosmetic industries. To meet the requirements for industrial application, the enzymes need to fulfill several criteria: (i) availability of sufficient quantities of proteins with constant quality and batch-to-batch reproducibility at low cost, (ii) broad substrate range, (iii) high stability under acidic pH and high solvent stability and (iv) activity at low temperatures (as the unselective chemical background reaction is significantly suppressed at low pH (< 4.5), low T and in the absence of water).

A number of (*R*)- and (*S*)-selective HNLs have been identified so far (Winkler et al., Comprehensive Chirality, 2012, 7, Synthetic Methods VI - Enzymatic and Semi Enzymatic, N. Turner, ed. 350-371). Currently, the (*R*)-selective hydroxynitrile lyases from *Prunus amygdalus* (*Pa*HNL), *Prunus mume* (*Pm*HNL), *Eriobotrya japonica Eⱼ*HNL, *Arabidobsis thaliana* (*At*HNL), *Linum usitatissimum* (*Lu*HNL) and several bacterial cupin HNLs, e. g. *Gt*HNL from *Granulicella tundricola,* can be heterologously expressed. Whereas *At*HNL, *Lu*HNL and cupin HNLs can be successfully expressed in *E. coli,* the glycosylated and disulfide bridge containing enzymes from *Rosaceae* e.g. *Pa*HNL, *Pm*HNL, and *Ej*HNL, can only be expressed with reasonable yield in *Pichia pastoris* as soluble and active protein.

Compared to other HNLs, wild-type cupin HNLs are less active (Hajnal et al., FEBS J. 2013 280(22):5815-28). *Gt*HNL catalyzed the synthesis of (*R*)-mandelonitrile from benzaldehyde and HCN with 80 % conversion and 90 % ee, which is not sufficient for industrial application.

In recent years, enzyme engineering by either random or directed methods was used to increase the activity and stability (at elevated temperature, at acidic or basic pH or in organic solvents), to broaden the substrate scope and to reverse the enantioselectivity of HNLs (Dadashipour and Asano, *ACS Catalysis 1*:1121-1149).

*At*HNL-WT resulted in 99% conversion of 3-phenylpropanal, but only 68% enantioselectivity after 22 h of reaction (however, using only 50 mM of aldehyde and 0.25 M HCN, Andexer et al., 2007).

The main disadvantage of the (*R*)-selective *At*HNL is its low stability at pH below 5.4. Recently, the stability of *At*HNL at acidic pH was improved by one pH unit exchanging eleven amino acids at its surface to the corresponding amino acids from the (*S*)-selective *Me*HNL, which belongs to the same fold and is more stable (Okrob et al., 2012. Chembiochem 13:797-802). However, the substrate scope is still rather limited.

The activity, enantioselectivity and substrate scope of *Pa*HNL was significantly improved by enzyme engineering (WO2008/071695).

### Summary of invention

It is the objective of the present invention to provide improved cupin-HNL enzymes which are capable to catalyze the asymmetric cyanohydrin reaction. It is a further objective of the present invention to modify cupin HNL to broaden their substrate scope.

It is a further objective of the present invention to provide an enhanced method for producing asymmetric cyanohydrin compounds. The method comprises the steps of providing an aldehyde or ketone compound and converting the compound to the corresponding asymmetric cyanohydrin compound in the presence of a cyanide donor and a cupin HNL. The invention relates in particular to a selective cupin HNL, which can enantioselectively catalyze the asymmetric cyanohydrin addition.

### Brief description of drawings

Fig. 1. Cartoon representation of one cupin monomer of the structure of *Gt*HNL (PDB-code: 4BIF).
Fig. 2: Specific activities of different *Gt*HNL variants at different pH and different buffers (100 mM citrate/phosphate or oxalate buffer at pH 4.5, 5 and 5.5) using 18 mM (*R*)-mandelonitrile as substrate. Depending on the activity of the variant different protein concentrations (50-200 µg of purified protein) were used. The increase of benzaldehyde was followed at 280 nm in a plate reader.
Fig. 3: Conversion of benzaldehyde (%) and *ee* (*R*) obtained with *Gt*HNL-WT (light grey), *Gt*HNL-V42T/Q110H (dark grey) and *Gt*HNL-A40H/V42T/Q110H (black) at different time-points. Conversions are shown as solid lines; the ees are shown as dashed lines.
Fig. 4: Protein sequence of *Gt*HNL (*Granulicella tundricola* MP45ACTX9, Uniprot E8WYN5)
Fig. 5: Protein sequence of *Gt*HNL triple mutant (A40H, V42T, Q110H)
Fig. 6: Protein sequence of *Ac*HNL (*Acidobacterium capsulatum*ATCC 51196; Uniprot C1F951),

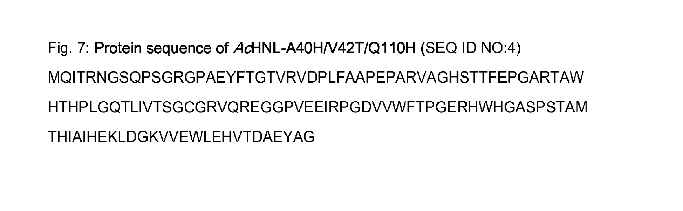

Fig. 7: Protein sequence of *Ac*HNL triple mutant (A40H, V42T, Q110H)

### Description of embodiments

In a first aspect, the invention relates to a mutated hydroxynitrile lyase (HNL) protein with a cupin fold, which is capable to catalyze the asymmetric cyanohydrin reaction, wherein at least one amino acid selected from the group consisting of alanine, valine and glutamine is substituted.

A further aspect of the invention is the cupin-HNL mutant protein as described above, wherein at least one amino acid at positions 40, 42 or 110 according to the numbering of SEQ ID NO:1 or SEQ ID NO:3 is substituted.

A further aspect of the invention is the cupin-HNL mutant protein as described above, wherein at least three amino acid at positions 40, 42 or 110 according to the numbering of SEQ ID NO:1 or SEQ ID NO:3 are substituted.

A further aspect of the invention is the cupin-HNL mutant protein as described above, characterized in that it comprises the amino acid sequence of the general formula:
(X1)(X2)(X3)(X4)F(X5)PGAR(X6)(X7)WH(X8)HP(X9)G, wherein
X1 is a A, V, L, F, Y, M, S, T, G, H, N or R residue, preferably it is an A
X2 is any amino acid preferably it is a T
X3 is a V, A, I, C, M, H, or T residue preferably it is a V
X4 is any amino acid preferably it is a T
X5 is any amino acid, preferably it is an E
X6 is a T, S or N residue, preferably it is a T
X7: is any amino acid, preferably it is an A
X8: is a T, S, or I residue, preferably it is a T
X9: is any amino acid, preferably it is an L
and wherein at least one of positions X1, or X3 is substituted by a H or T residue.

A further aspect of the invention is the cupin-HNL mutant protein as described above, wherein the cupin-HNL is at least 85%, or at least 90%, or at least 95%, or at least 98% identical to the amino acid sequence of the respective wild type enzyme.

According to the invention, the term "modification" means a deletion or substitution or insertion of at least one amino acid. Specifically, the modification is a substitution of one amino acid.

A further aspect of the invention is a cupin-HNL as described above, comprising at least one, specifically at least two, specifically at least three amino acids, specifically at least four, specifically at least five or more modifications.

In a specific embodiment of the invention, the cupin-HNL comprises one, two or three amino acid substitutions.

According to the embodiment of the invention, any amino acid can be selected to substitute the amino acid of the wild type sequence.

Specifically, said substituted amino acids are selected from histidine and threonine.

In a further aspect, the invention relates to a cupin hydroxynitrile lyase as described above, wherein the amino acid sequence of the cupin-HNL is at least 85%, at least 86%, at least 87%, at least 88%, at least 89%, at least 90%, at least 91%, at least 92%, at least 93%, at least 94%, at least 95%, at least 96%, at least 97%, or at least 98% identical to the respective wild type enzyme.

In a further aspect, the invention relates to a cupin-HNL as described above, which is modified at any one of positions 40, 42 and/or 110 according to the numbering of SEQ ID NO:1 or SEQ ID NO:3.

According to the embodiment of the invention, the modification at any of positions 40, 42 and/or 110 are amino acid substitutions. According to a further embodiment, the modified cupin-HNL of the invention contains one, two or three substitutions at any of positions 40, 42 and 110.

In a further aspect, the invention relates to a cupin-HNL as described above, having the SEQ ID NO:2 or SEQ ID NO:4.

According to a specific embodiment, the modified cupin-HNL is A40H V42T Q110H according to the numbering of SEQ ID NO:2.

A further aspect of the invention is the cupin-HNL mutant protein as described above, having the SEQ ID NO:2 or SEQ ID NO:4.

A further aspect of the invention is an isolated polynucleic acid molecule encoding a cupin-HNL as described above.

A further aspect of the invention is a vector comprising an isolated DNA molecule as described above.

A further aspect of the invention is a recombinant non-human cell obtained by introducing the vector as described above.

A further aspect of the invention is a culture obtained by culturing the recombinant cell as described above.

A further aspect of the invention is a cupin-HNL recovered from the culture as described above.

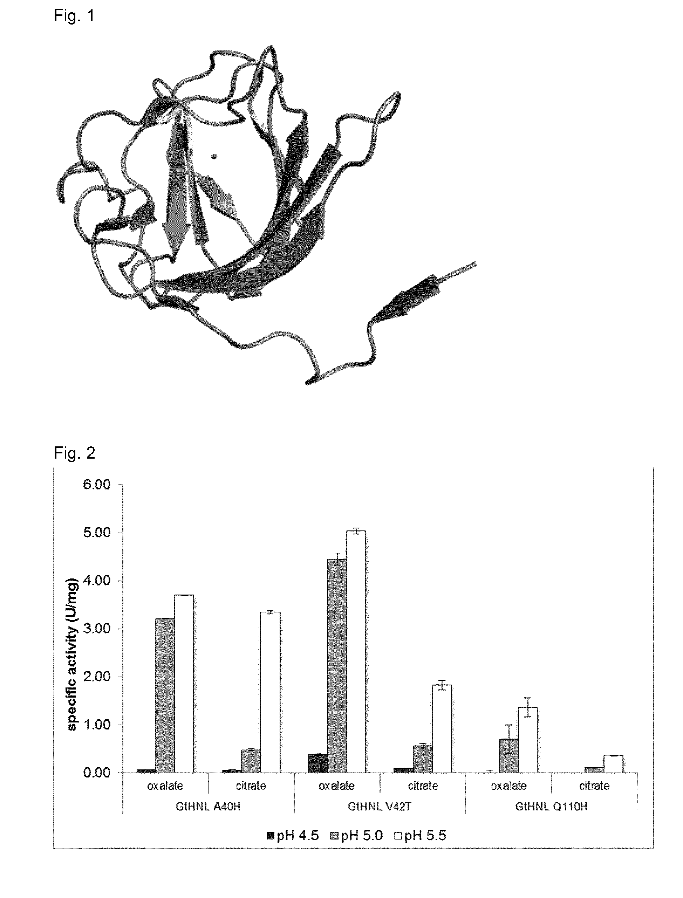

Proteins of the cupin superfamily of proteins (PFAM: CL0029) contain a conserved beta barrel domain composed of 10 to 12 anti-parallel beta-strands. Cupa is the latin term for barrel. In the structural classification of proteins database (SCOP) they are classified as the RmIC-like cupins superfamily [51182] within the doublestranded β-helix fold.The cupin fold is found in a wide variety of enzymes, but also non-enzymatic proteins. The cupin domain can be found once, twice or more in a protein structure, either alone or in combination with other domains. Although proteins in the cupin superfamily show very low overall sequence similarity, they all contain two short but partially conserved cupin sequence motifs separated by a less conserved intermotif region that varies both in length and amino acid sequence (Fig. 1). Proteins in the cupin superfamily have a wide range of biological functions in archaea, bacteria and eukaryotes.

Cupins are structurally conserved and usually contain two conserved motifs, G-(X)₅-H-X-H-(X)_{3,4}-E-(X)₆-G (motif 1) and G-(X)₅-P-X-G-(X)₂-H-(X)₃-N (motif 2). The two motifs also include the residues for metal binding. Most cupins are metal-binding proteins that bind divalent cations such as iron, zinc, manganese, copper, nickel or cadmium. The metal is usually involved in the enzymatic reaction either directly in the reaction mechanism or at least via an interaction with the substrate.

The HNL from *Granulicella tundricola* (*Gt*HNL) was shown to catalyse the (*R*)-selective synthesis of (*R*)-mandelonitrile with a very good conversion of 80% and an enantiomeric excess of 90% in a biphasic reaction system (Hajnal et al., 2013). But unfortunately the substrate scope was quite limited. Moreover, the enzyme exhibited a specific activity of only 1.74 U/mg at pH 6.0 in sodium oxalate buffer for the cleavage of (*R*)-mandelonitrile, which is low compared to other HNLs in literature.

Variants were prepared by site-saturation mutagenesis of the cupin-HNL gene. Site-saturation mutagenesis of the *cupin-HNL* may be performed with Single-Primer Reactions IN Parallel (SPRINP) mutagenesis protocol (Edelheit et al., BMC Biotechnol. 2009 Jun 30;9:61), an adapted QuikChange protocol (e.g. Zheng et al., Nucleic Acids Res.; 2004;32(14):e115; Liu and Naismith, BMC Biotechnol. 2008;8:91) or using the QuikChange Site Directed Mutagenesis Kit (from Stratagene) or a comparable kit from another company according to the manufacturer's instructions or by other conventional methods as described for example in Current Protocols in Molecular Biology, Ausubel et al., 2004). At the amino acid level, these modifications result in replacement of one amino acid residue with another amino acid residue.

Site-saturation libraries were screened on colony level with the filter assay established by Krammer et al. (J. Biotechnol. 2007;129(1):151-61).

The amino acid residues in cupin-HNL variants having improved properties and obtained by said screening, corresponding to position 40, 42 and position 110 of the mature wild type *Gt*HNL protein of (SEQ ID NO:1), are replaced with a different amino acid, for example with arginine, histidine, threonine, asparagine, or tyrosine.

The amino acid residues in cupin-HNLs discovered in the said screening corresponding to position 40, 42 and position 110 of the mature wild type *Gt*HNL protein of (SEQ ID NO:1) are transferred to another cupin HNL from *Acidobacterium capsulatum* ATCC 51196 (SEQ ID NO:2).

A further aspect of the invention is a method for producing enantiopure cyanohydrin compounds, wherein an aldehyde or ketone compound is converted to the corresponding cyanohydrin compound in the presence of a cyanide donor and a cupin-HNL as described above.

A further aspect of the invention is a method as described above, wherein a compound of general formula I is reacted with a compound of general formula II in the presence of a cupin-HNL to yield an enantiopure cyanohydrin compound III 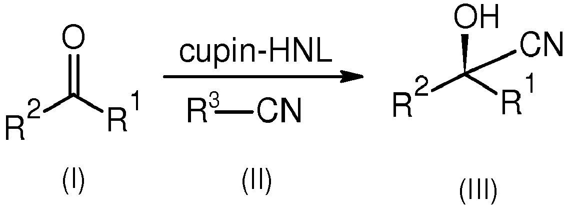 wherein
R¹ and R² are independently from one another H, C₁₋₂₀alkyl, C₂₋₂₀alkenyl, C₂₋₂₀alkynyl, C₃₋₁₀cycloalkyl, C₄₋₂₀cycloalkylalkyl, C₆₋₁₄aryl, C₇₋₂₀arylalkyl, 3-14 membered heterocycloalkyl, 4-20 membered heterocycloalkylalkyl, 5-20 membered heteroaryl or 6-20 membered heteroarylalkyl, optionally substituted by one or more R^{a};
R³ is H, an alkali metal, C(CH₃)₂OH, or C₁₋₂₀alkyl; and
each R^{a} is independently H, halogen, -CF₃, -OR^{b}, -NR^{b}R^{b}, -(CH₂)ₙCOOR^{b}, -(CH₂)ₙC(=O)R^{b}, -(CH₂)ₙCONR^{b}R^{b}, C₁₋₂₀alkyl, C₂₋₂₀alkenyl,or C₂₋₂₀alkynyl; and
each R^{b} is independently H or optionally substituted C₁₋₂₀alkyl, C₂₋₂₀alkenyl, or C₂₋₂₀alkynyl; and
n is 0, 1, 2 or 3.

An alkyl group, if not stated otherwise, denotes a linear or branched C₁₋₂₀alkyl, preferably a linear or branched chain of one to twenty carbon atoms.

An alkenyl group, if not stated otherwise, denotes a partially unsaturated linear or branched C₂₋₂₀alkenyl, preferably a linear or branched chain of two to twenty carbon atoms that contains at least one double bond.

An alkynyl group, if not stated otherwise, denotes a partially unsaturated linear or branched C₂₋₂₀alkynyl, preferably a linear or branched chain of two to twenty carbon atoms that contains at least one triple bond.

A cycloalkyl group denotes a monocyclic non-aromatic hydrocarbon ring containing three to ten carbon atoms, preferably four to six carbon atoms, or a bicyclic non-aromatic hydrocarbon ring system containing seven to ten carbon atoms, preferably eight to ten carbon atoms, wherein the cycloalkyl group optionally comprises one or more double bonds.

A heterocycloalkyl group denotes a monocyclic non-aromatic hydrocarbon ring containing three to fourteen carbon atoms, preferably four to eight carbon atoms, or a bicyclic non-aromatic hydrocarbon ring system containing seven to fourteen carbon atoms, preferably eight to ten carbon atoms, wherein in the heterocycloalkyl group one or more of the carbon atoms of the hydrocarbon ring or ring system is replaced by a group selected from the group comprising -N-, -O-, -S-, -S(O)-, -S(O)₂-, -Si- and -P-; wherein the heterocycloalkyl group optionally comprises one or more double or triple bonds.

An aryl group preferably denotes a mono- or bicyclic, preferably monocyclic aromatic hydrocarbon group having six to fourteen carbon atoms; the aryl group is preferably phenyl.

A heteroaryl group denotes an aromatic 5- or a 6- membered monocyclic hydrocarbon group wherein at least one of the carbon atoms is replaced by a heteroatom like O, N, and/or S, and wherein the aromatic monocyclic 5- or 6-membered cyclic hydrocarbon group is optionally fused to a further monocyclic 5- to 7-membered, preferably 5- or 6-membered, aromatic or nonaromatic hydrocarbon ring, wherein in the further monocyclic aromatic or nonaromatic hydrocarbon ring one or more, preferably one or two carbon atoms may be replaced by a heteroatom like O, N, and/or S.

A halogen group is chlorine, bromine, fluorine or iodine.

The method according to the invention can be carried out in a mono- or biphasic system or in an emulsion.

The monophasic reaction solution comprises an aqueous or an organic solvent.

Appropriate aqueous solutions are for example water, a cupin hydroxynitrile lyase containing solution, or a buffer solution. Examples for buffer solutions are phosphate buffer, citrate buffer, acetate buffer, borate buffer, MES, HEPES, Tris buffer, or mixtures thereof. The pH of these solutions can be between pH 2 and 8, preferably from 2 to 5.

Appropriate organic solutions can be slightly water-miscible or water immiscible aliphatic or aromatic hydrocarbons, which are optionally halogenated, alcohols, ethers or esters or mixture thereof or the substrate itself. Suitable are for example, but not limited to ethyl acetate, butyl acetate, methyl *tert*-butyl ether, diisopropyl ether, dibutyl ether, carbon tetrachloride, benzene, toluene, cyclohexane, hexadecane, hexane, heptane, chloroform, xylene, pentanol, hexanol, octanol and dodecanol, benzaldehyde, or mixtures thereof. Also applicable are neoteric solvents.

The advantages of conducting bioconversions in aqueous - organic solvent two-liquid phase systems are well known in the art. The biphasic system consists of two phases mutually not miscible, e.g. an aqueous and an organic phase.

A further aspect of the invention is the method as described above, wherein the reaction is carried out in a mono- or biphasic system or in an emulsion.

Thus, in a further aspect the invention relates to a method as described above, wherein the biphasic system comprises aqueous and organic solution as described herein.

The conversion moreover takes place at temperatures of from -10°C to +50°C, preferably at 0°C to 25°C.

The choice of applicable electrophiles ranges from aromatic to heteroaromatic and aliphatic aldehydes. Depending on the substrate and reaction systems yields up to 99% or enantiomeric excess > 99% could be obtained by the inventive method.

A further aspect of the invention is the method as described above, wherein the cupin-HNL is a purified enzyme or whole cell suspension or contained in cleared lysate.

A further aspect of the invention is the method as described above, wherein the asymmetric cyanohydrin compound is obtained with at least 95%, preferably with at least 98%, more preferred with at least 99% enantiomeric excess (e.e.).

A further aspect of the invention is the method as described above, wherein the asymmetric cyanohydrin compound is obtained with a conversion rate of at least 15%, preferably with at least 20%, more preferred with at least 50%.

In another aspect, the invention relates to the method as described above, wherein the cupin-HNL is a purified enzyme or contained in whole cell or in a cleared lysate or in immobilized form, for example on a support such as Celite®, Avicel, etc. or as cross-linked enzyme aggregate (CLEA).

The Examples which follow are set forth to aid in the understanding of the invention but are not intended to, and should not be construed to limit the scope of the invention in any way. The Examples do not include detailed descriptions of conventional methods, e.g., cloning, transfection, and basic aspects of methods for overexpressing proteins in microbial host cells. Such methods are well known to those of ordinary skill in the art.

### Examples

### Example 1: Introduction and identification of advantageous mutations

Site-saturation mutagenesis at eight active site positions (Ala40, Val42, Phe44, Thr50, Leu61, His96, His106 and Glu110) of the *Gt*HNL gene (gene ID: NC_012483, codon-optimized for *E. coli*) from *Granulicella tundricula* was performed with Single-Primer Reactions IN Parallel (SPRINP) mutagenesis protocol (Edelheit et al., 2009).

Cultivation of the mutant libraries was performed according to standard procedures. The screening of the libraries was performed with a colorimetric colony-based HNL filter assay (Krammer et al., 2007). Improved variants were obtained in the libraries of position A40, V42 and Q110.

### Example 2: Protein production

*E. coli* BL21-Gold(DE3) was used as expression host (Stratagene, La Jolla, CA, USA). The cells were grown in LB (lysogeny broth, Lennox) medium (Carl Roth GmbH, Karlsruhe, Germany) supplemented with kanamycin sulphate (40 mg/L final concentration). Expression of recombinant protein was initiated by addition of 0.1 mM IPTG (isopropyl β-D-1-thiogalactopyranoside) to OD600 ~0.8 cultures, and cultivation was continued at 25°C for 20 h. As it was shown before that the HNL reaction catalyzed by *Gt*HNL is manganese-dependent, all enzymes were grown with manganese present in the expression medium. Routinely, 100 µM of MnCl₂ was added concomitantly with the induction except for controls, which should not contain MnCl₂. If needed, the successful incorporation was confirmed by inductively coupled plasma/optical emission spectroscopy (ICP-OES). The cells were harvested, resuspended in cold buffer (10 mM sodium phosphate buffer pH 7.0) and disrupted by sonification (Branson Sonifier S-250, set to 80% duty cycle, and output control 7) two times for 3 min, cooled on ice. The cell lysate was centrifuged for an hour at 50,000 g to remove unbroken cells and insoluble material. The cell free lysate was filtered through a 0.45 µm syringe filter and concentrated to about 40-50 mg/ml using Vivaspin 20 Centrifugal Filter Units (10,000 Da molecular-weight cut-off; Sartorius). The concentrated lysates were aliquoted and frozen until further use. Protein expression was monitored by standard SDS-PAGE. *Gt*HNL and all variants thereof were expressed in high yield as soluble protein in *E. coli* BL21-Gold(DE3) reaching yields of >50% of total soluble protein.

The purification procedure was adapted from a protocol published for *Gt*HNL applying anion-exchange chromatography and size exclusion chromatography (Hajnal et al., 2013). Cell pellets were resuspended Buffer A (50 mM Bis-Tris/HCl, pH 6.8 or 6.9, 50 mM NaCl), and the cleared lysates were loaded on a QSepharose anion-exchange column (HiTrapTM Q FF 5 mL, GE Healthcare, Uppsala, Sweden). The proteins were eluted with 10% buffer B (50 mM Bis-Tris/HCl, pH 6.8 or 6.9, 1 M NaCl). If needed, size exclusion chromatography was performed on a Superdex 75 HiLoad 16/600 column (GE Healthcare, Uppsala, Sweden) pre-equilibrated with 50 mM NaPi, pH 7.0, 100 mM NaCl. Fractions containing the proteins of interest were pooled, and brought to a protein concentration of 10-20 mg/mL using Vivaspin 20 Centrifugal Filter Units.
Protein concentrations of cell free lysates were routinely determined using the Bradford assay (Biorad, Hercules, CA, USA). Concentrations of purified proteins were determined with a Nanodrop spectrophotometer (model 2000c, Peqlab, Erlangen, Germany) at 280 nm applying an extinction coefficient calculated based on the amino acid sequence using Protparam. The protein was stored at -80°C or -20°C until further use.

### Example 3: Cyanogenesis of (R)-mandelonitrile

The activity of *Gt*HNL and several promising variants in the cyanogenesis of (*R*)-mandelonitrile was determined in two different buffer systems (citrate/ phosphate and oxalate) at different pH values (4.0, 4.5, 5.0, 5.5) spectrophotometrically by following the increase of benzaldehyde absorption at 280 nm. A standard substrate concentration of 18 mM (*R*)-mandelonitrile and different protein concentrations between 5 µg/mL and 1 mg/mL (depending on the activity of the variant) were used.

One mandelonitrile cyanogenesis unit is defined as the amount of enzyme that catalyzes the formation of 1 µmol benzaldehyde from mandelonitrile dissolved in aqueous buffer in 1 min at 25°C.

The specific activities of the different variants are displayed in Fig. 2 and Table 1.

**Table 1. Specific activities (U/mg) related to Figure 2.**

| | buffer | | | | | |
|---|---|---|---|---|---|---|
| | oxalate | | | citrate | | |
| *Gt*HNL-variant | pH 4.5 | pH 5.0 | pH 5.5 | pH 4.5 | pH 5.0 | pH 5.5 |
| WT | 0.03 | 0.2 | 0.29 | 0.04 | 0.12 | 0.28 |
| A40H | 0.07 | 3.21 | 3.69 | 0.06 | 0.48 | 3.34 |
| V42T | 0.37 | 4.45 | 5.03 | 0.10 | 0.57 | 1.83 |
| Q110H | 0.00 | 0.70 | 1.37 | 0.00 | 0.11 | 0.35 |

All chosen variants indeed showed a higher activity than *Gt*HNL-WT, especially *Gt*HNL-A40H and V42T display an improvement of the activity by ~ 13 and 17 times, respectively, in the clearly preferred sodium oxalate buffer at pH 5.5.

### Example 4: (R)-mandelonitrile synthesis

*Gt*HNL and several variants were tested for their ability to catalyze the cyanohydrin synthesis from benzaldehyde (1a) and HCN.

All reactions involving cyanides were performed in a well ventilated hood equipped with an HCN detector (Drager PACIII; Lübeck, Germany). The reactions were performed in a two-phase system with 1 mL organic phase containing the substrate (0.5 M aldehyde, 1a was freshly distilled prior to use), the internal standard (0.2 % v/v triisopropylbenzene) and 2 M HCN in methyl *tert*-butyl ether (MTBE). The 500 µL aqueous phase comprised the enzyme (cleared lysate with a total protein content of 45 mg/mL containing ~ 50% of cupin (11.25 mg of total cupin protein in the reaction), in 100 mM sodium acetate buffer, pH 4. HCN was produced and extracted in MTBE as described before by Okrob et al. (2011). The reactions were performed at 1,000 rpm and 5°C. Samples were analyzed by chiral HPLC.

First experiments for the synthesis of (*R*)-mandelonitrile using cleared lysate of *E. coli* expressing the most promising variants of *Gt*HNL showed that most variants displayed improved conversion and/or enantioselectivity compared to *Gt*HNL-WT (Table 2).

**Table 2. Conversion of benzaldehyde (%) and ee (R) obtained with site-directed GtHNL mutants after 24 h.**

| *Gt*HNL-varian*t* | conversion (%) | *ee* (%) |
|---|---|---|
| WT | 84.6 | 88.7 |
| A40H | 99.0 | 87.9 |
| A40R | 97.6 | 94.5 |
| Q110H | 95.4 | 95.4 |
| V42T | 98.8 | 96.3 |

In comparison to the wild type, *Gt*HNL-A40H showed a significantly increased conversion (99%), and a similar ee (87.9 %). *Gt*HNL-A40R exhibited improved conversion and *ee* values. Also the exchange of valine 42 to threonine and glutamine 110 to histidine improved both, conversion and *ee.*

### Example 5: Combination and characterization of advantageous mutations

The advantageous mutations (A40H, A40R, V42T and Q110H) identified above were combined as double and triple mutants by site-directed mutagenesis. The variants were produced as described in example two and tested in the synthesis (as described in Example 4) and cyanogenesis (as described in Example 3) of (*R*)-mandelonitrile.

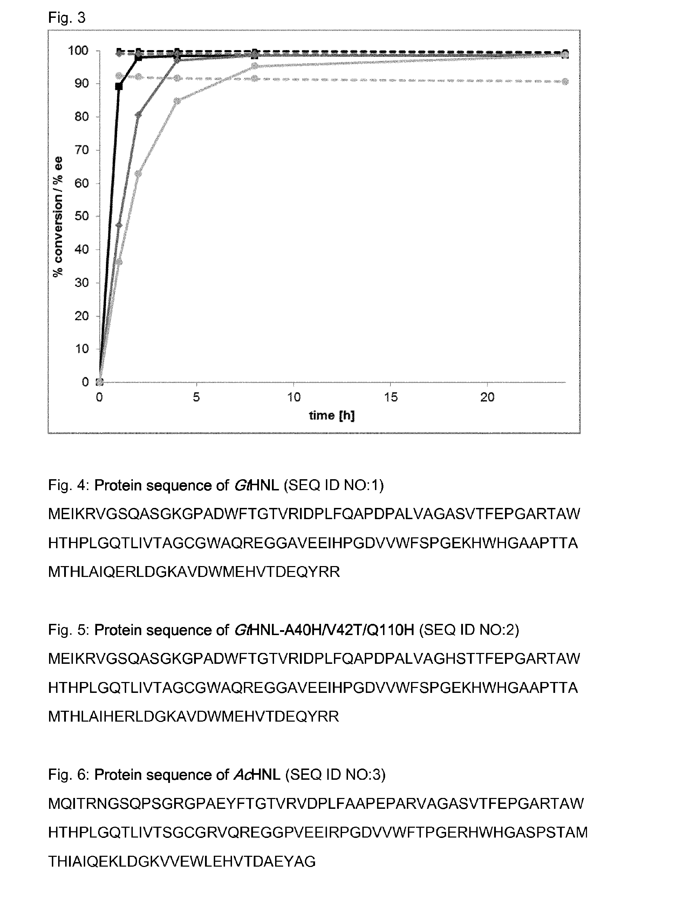

The two-phase system consisted of 500 µL of lysate (11.25 mg of total *Gt*HNL-variant protein in 100 mM Na-acetate, pH 4.0) as aqueous phase and 1 mL MTBE containing 0.5 M benzaldehyde and 2 M HCN as organic phase, 1000 rpm, 5°C. The best double mutant *Gt*HNL-V42T/Q110H exhibited 98.7% conversion of benzaldehyde and *ee* after 24 h (80.6 % conversion with 99% *ee* after 2 h, Table 3 and Fig. 3), which is a further improvement compared to the single variants. The rest of the double mutants showed only a slight or even no improvement in the synthesis reaction.

**Table 3. Conversion and enantioselectivity of GtHNL variants related to Figure 3.**

| | | Reaction time | | | | |
|---|---|---|---|---|---|---|
| *Gt*HNL-variant | | 1 h | 2 h | 4 h | 8 h | 24 h |
| WT | conv (%) | 36.2 | 62-8 | 84.8 | 95.3 | 98.6 |
| | *ee* (%) | 92.4 | 92.0 | 91.7 | 91.5 | 90.7 |
| V42TQ110H | conv (%) | 47.4 | 80.6 | 97.1 | 98.5 | 98.7 |
| | *ee* (%) | 99.1 | 99.0 | 99.1 | 99.0 | 98.7 |
| A40HV42TQ110H | conv (%) | 89.2 | 98.0 | 98.4 | 98.5 | 98.6 |
| | *ee* (%) | > 99.9 | 99.9 | 99.9 | 99.8 | 99.5 |

Most interestingly however, was the result obtained for the triple mutant *Gt*HNL-A40H/V42T/Q110H, which showed nearly full conversion after 2 h, with an *ee* (*R*) of > 99.9 % (Table 5 and Fig. 3).

Purified *Gt*HNL-A40H/V42T/Q110H showed a specific activity of 137±10 U/mg in the cyanogenesis of (*R*)-mandelonitrile in citrate/phosphate buffer pH 5.5, which corresponds to a ~ 490-fold increase of activity compared to *Gt*HNL-WT at the same reaction conditions. In contrast to GtHNL-WT residual activity was still detected a pH 4 (2.3±0.04 U/mg).

### Example 6: Substrate screening

Purified *Gt*HNL-A40H/V42T/Q110H was subjected to a substrate screening including several aldehydes (Table 4). The reactions were performed in a two-phase system consisting of 500 µL of purified enzyme (4.7 mg of total protein in 100 mM Na-acetate, pH 4.0) as aqueous phase and 1 mL MTBE containing 0.5 M aldehyde (1a: benzaldehyde, 2a: 2-chloro-benzaldehyde, 3a: 3-phenylpropanal, 4a: 3-phenylprop-2-enal, 5a: 2-furfural) and 2 M HCN as organic phase, 1,000 rpm, 5°C. The samples were analyzed by chiral GC.

**Table 4. Conversion (%) and enantioselectivity (%) of purified GtHNL-A40H/V42T/Q110H and GtHNL-WT towards different aldehydes.**

| | Reaction time | | | | | | | |
|---|---|---|---|---|---|---|---|---|
| | 2 h | | | | 24 h | | | |
| GtHNL-variant | A40H/V42T/Q110H | | WT | | A40H/V42T/Q110H | | WT | |
| Substrate | conv (%) | *ee* (%) | conv (%) | *ee* (%) | conv (%) | *ee* (%) | conv (%) | *ee* (%) |
| 1a | 87.9 | > 99.9 | n.d. | n.d. | 98.6 | 99.8 | n.d. | n.d. |
| 2a | 62.1 | 99.1 | 24.5 | 14.9 | 99.9 | 99.4 | 60.5 | 23.8 |
| 3a | 85.4 | 97.9 | 26.4 | -13.9 | 94.7 | 98.5 | 78.9 | -13.5 |
| 4a | 70.7 | > 99.9 | 17.4 | > 99.9 | 97.0 | > 99.9 | 21.8 | 77.8 |
| 5a | 98.2 | 99.5 | 77.9 | 74.8 | 98.3 | 98.1 | 95.8 | 72.2 |

| | | | | | | | | |
|---|---|---|---|---|---|---|---|---|
| n.d. not determined | | | | | | | | |

For all these substrates excellent enantioselectivities and good to excellent conversions could be achieved with *Gt*HNL-A40H/V42T/Q110H. Reducing the applied amount of enzyme to a tenth (from 4.7 mg to 0.5 mg of total protein) still resulted in excellent enantioselectivities, reaching full or almost full conversion after 24 h (Table 5).

**Table 5. Conversion (%) and enantioselectivity (%) of purified GtHNL-A40H/V42T/Q110H towards different aldehydes applying different enzyme to substrate ratios.**

| | | Reaction time | | | |
|---|---|---|---|---|---|
| substrate | mg enzyme / mmol substrate | 2 h | | 24 h | |
| | | conv (%) | ee (%) | conv (%) | ee (%) |
| 1a | 9.4 | 87.9 | > 99.9 | 98.6 | 99.8 |
| | 4.9 | 95.8 | 99.7 | 97.9 | 99.6 |
| | 1 | 77.4 | 99.3 | 99.4 | 99.0 |
| 2a | 9.4 | 62.1 | 99.1 | 99.9 | 99.4 |
| | 4.9 | 89.1 | 97.7 | 99.9 | 98.4 |
| | 1 | 60.6 | 93.2 | > 99.9 | 96.1 |
| 3a | 9.4 | 85.4 | 97.9 | 94.7 | 98.5 |
| | 4.9 | 90.6 | 97.2 | 97.1 | 98.2 |
| 5a | 9.4 | 98.2 | 99.5 | 98.3 | 98.1 |
| | 4.9 | 96.0 | 99.5 | 97.7 | 98.2 |

### Example 7: Transfer of advantageous mutations to AcHNL

The advantageous mutations A40H and V42T and Q110H were successfully transferred as single and triple mutations to a cupin HNL from *Acidobacterium capsulatum* ATCC 51196 (*Ac*HNL) by site-directed mutagenesis. *Ac*HNL and its variants were produced as described for *Gt*HNL and its variants (see Example 2) and tested in the cyanogenesis (as described in Example 3) and synthesis (as described in Example 4) of (*R*)-mandelonitrile.

The activity of purified *Ac*HNL and mutants thereof was examined in the cyanogenesis of 18 mM (*R*)-mandelonitrile in 0.1 M citrate phosphate buffer, pH 4.5, 5.0 and 5.5; 0.1 M sodium oxalate buffer, pH 4.5, 5.0 and 5.5). Purified *Ac*HNL-A40H/V42T/Q110H displayed a specific activity of 139±11.2 U/mg in the cyanogenesis of (*R*)-mandelonitrile in citrate/phosphate buffer pH 5.5 (Table 6), which is almost identical to *Gt*HNL-A40H/V42T/Q110H.

**Table 6. Specific activities (U/mg) of AcHNL and mutants thereof for (R)-mandelonitrile**

| | buffer | | | | | |
|---|---|---|---|---|---|---|
| | oxalate | | | citrate | | |
| *Ac*HNL-variant | pH 4.5 | pH 5.0 | pH 5.5 | pH 4.5 | pH 5.0 | pH 5.5 |
| WT | 0.12 | 0.35 | 0.48 | 0.03 | 0.09 | 0.3 |
| A40H | n.d. | 4.27 | 6.56 | 0.12 | 0.73 | 5.85 |
| V42T | 0.11 | 2.26 | 3.51 | 0.15 | 0.65 | 2.55 |
| Q110H | 0.007 | 0.90 | 1.65 | 0.03 | 0.09 | 0.36 |
| A40H/V42T/Q110T | n.d. | 58.31 | 82.87 | 4.50 | 32.00 | 139.21 |

| | | | | | | |
|---|---|---|---|---|---|---|
| n.d. not determined | | | | | | |

The synthesis reactions were performed in a two-phase system consisting of 500 µL of purified enzyme (~ 4.0 mg of total protein in 100 mM Na-acetate, pH 4.0) as aqueous phase and 1 mL MTBE containing 0.5 M benzaldehyde and 2 M HCN as organic phase, 1,000 rpm, 5°C (Table 7). *Ac*HNL-A40H/V42T/Q110H showed 99.4 % conversion of benzaldehyde with 99.7 % *ee* (*R*) after 24 h.

**Table 7. Conversion of benzaldehyde (%) and ee (%) obtained by AcHNL variants after 2 and 24 h.**

| | Reaction time | | | |
|---|---|---|---|---|
| | 2 h | | 24 h | |
| *Ac*HNL-Variant | Conv (%) | *ee* (%) | Conv (%) | *ee* (%) |
| WT | 23.8 | 90.3 | 75.6 | 88.5 |
| A40H | 41.0 | 96.5 | 97.3 | 96.1 |
| V42T | 28.3 | 91.5 | 75.5 | 88.3 |
| Q110H | 41.6 | 95.8 | 87.4 | 93.7 |
| A40H/V42T/Q110H | 91.8 | 99.7 | 99.4 | 99.7 |

The work leading to this invention has received funding from the European Union's Seventh Framework Program (FP7/2007-2013) under grant agreement n° 289646.

## Claims

1. A mutated cupin-hydroxynitrile lyase (HNL) protein, which is capable to catalyze the asymmetric cyanohydrin reaction, wherein at least one amino acid selected from the group consisting of alanine, valine and glutamine is substituted.

2. The cupin-HNL mutant protein according to claim 1, wherein at least one amino acid at positions 40, 42 or 110 according to the numbering of SEQ ID No.1 is substituted.

3. The cupin-HNL mutant protein according to claim 1 or 2, wherein at least three amino acid at positions 40, 42 or 110 according to the numbering of SEQ ID No.1 are substituted.

4. The cupin-HNL mutant protein according to claim 1 **characterized in that** it comprises the amino acid sequence of the general formula:
(X1)(X2)(X3)(X4)F(X5)PGAR(X6)(X7)WH(X8)HP(X9)G, wherein
X1 is a A, V, L, F, Y, M, S, T, G, H, N or R residue, preferably it is an A
X2 is any amino acid preferably it is a T
X3 is a V, A, I, C, M, H, or T residue preferably it is a V
X4 is any amino acid preferably it is a T
X5 is any amino acid, preferably it is an E
X6 is a T, S or N residue, preferably it is a T
X7: is any amino acid, preferably it is an A
X8: is a T, S, or I residues, preferably it is a T
X9: is any amino acid preferably it is an L
and wherein at least one of positions X1, or X3 is substituted by a H or T residue.

5. The cupin-HNL according to claim 1 or 2, wherein the cupin-HNL is at least 85%, or at least 90%, or at least 95%, or at least 98% identical to the amino acid sequence of the respective wild type enzyme.

6. The cupin-HNL according to claim 1, having the SEQ ID NO:2 or SEQ ID NO:4.

7. An isolated polynucleic acid molecule encoding a cupin-HNL according to any one of claims 1 to 6.

8. A vector comprising an isolated DNA molecule of claim 7.

9. A recombinant non-human cell obtained by introducing the vector of claim 8.

10. A culture obtained by culturing the recombinant cell of claim 9.

11. A cupin-HNL recovered from the culture of claim 10.

12. A method for producing enantiopure cyanohydrin compounds, wherein an aldehyde or ketone compound is converted to the corresponding cyanohydrin compound in the presence of a cyanide donor and a cupin-HNL according to any one of claims 1 to 6.

13. The method according to claim 12, wherein a compound of general formula I is reacted with a compound of general formula II in the presence of a cupin-HNL to yield an enantiopure cyanohydrin compound III 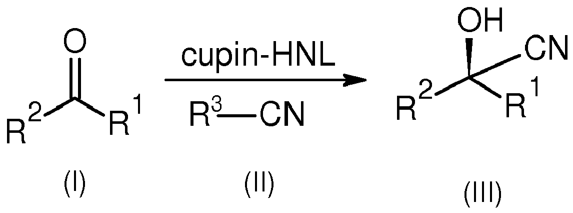 wherein
R¹ and R² are independently from one another H, C₁₋₂₀alkyl, C₂₋₂₀alkenyl, C₂₋₂₀alkynyl, C₃₋₁₀cycloalkyl, C₄₋₂₀cycloalkylalkyl, C₆₋₁₄aryl, C₇₋₂₀arylalkyl, 3-14 membered heterocycloalkyl, 4-20 membered heterocycloalkylalkyl, 5-20 membered heteroaryl or 6-20 membered heteroarylalkyl, optionally substituted by one or more Ra;
R³ is H, an alkali metal, C(CH₃)₂OH, or C₁₋₂₀alkyl; and
each R^{a} is independently H, halogen, -CF₃, -OR^{b}, -NR^{b}R^{b}, -(CH₂)ₙCOOR^{b}, -(CH₂)ₙC(=O)R^{b}, -(CH₂)ₙCONR^{b}R^{b}, C₁₋₂₀alkyl, C₂₋₂₀alkenyl,or C₂₋₂₀alkynyl; and each R^{b} is independently H or optionally substituted C₁₋₂₀alkyl, C₂₋₂₀alkenyl, or C₂₋₂₀alkynyl; and
n is 0, 1, 2 or 3.

14. The method according to claim 12 or 13, wherein the reaction is carried out in a mono- or biphasic system or in an emulsion.

15. The method according to any one of claims 12 to 14, wherein the cupin-HNL is a purified enzyme or a whole cell suspension or contained in cleared lysate.
